# EUROPEAN PATENT APPLICATION

(11) **EP 2 226 018 A1**
(43) Date of publication of application: **08.09.2010**
(21) Application number: 10006846.9
(22) Date of filing: 26.03.2002
(51) Int. Cl.: A61B 17/04

(54) **Oil coated sutures**

(30) Priority: 26.03.2001 US 278687 P
(62) Divisional of application: 02731176.0
(71) Applicant: Tyco Healthcare Group, LP, New Haven CT 06511 (US)
(72) Inventor: Roby, Mark, Killingworth CT 06419 (US); Kennedy, John, Guilford CT 06437 (US); Stevenon, Richard, Colchester CT 06415 (US)
(74) Representative: Soames, Candida Jane

(57) **Abstract**

A suture comprising: a filament made from a synthetic, non-absorbable polymer composition; and a coating, the coating being made from a composition containing an oil selected from the group consisting of castor oil and mineral oil.

## Description

### BACKGROUND

### 1. Technical Field

The present invention relates generally to coatings for filaments. More particularly, the present invention relates to oil coatings for filaments or sutures.

### 2. Background of Related Art

Many synthetic materials are presently used as surgical sutures. These materials may be used as single filament strands, i.e., monofilament sutures, or as multifilament strands in a braided, twisted or other multifilament construction. Synthetic sutures have been made from materials such as polypropylene, nylon, polyamide, polyethylene, polyesters such as polyethylene terephthalate, and segmented polyether-ester block copolymers. In addition, absorbable synthetic sutures have been prepared from synthetic polymers such as polymers containing glycolide, lactide, dioxanone and/or trimethylene carbonate. Natural materials have also been used to make sutures. For example, silk has been used to make non-absorbable sutures. As another example, catgut sutures are absorbable sutures made from a natural material.

Sutures intended for the repair of body tissues must meet certain requirements: they must be non-toxic, capable of being readily sterilized, they must have good tensile strength and have acceptable knot-tying and knot characteristics. The sutures should also be sufficiently durable from the point of view of fray resistance.

### SUMMARY

It has now been found that a suture formed from one or more filaments and coated with an oil, such as for example, mineral oil or castor oil, exhibits good durability as reflected by fray resistance. In another aspect, the present invention embraces a method for improving the handling characteristics of a suture by applying to the suture a coating comprising an oil. Preferred coatings comprise castor oil or mineral oil.

### DESCRIPTION OF PREFERRED EMBODIMENTS

Sutures in accordance with the present invention are prepared by applying a coating to one or more filaments. Preferably, the suture is made from a synthetic material. Suitable synthetic materials include, but are not limited to polypropylene, nylon, polyamide, polyethylene, polyesters such as polyethylene terephthalate, segmented polyether-ester block copolymers and polyurethanes. When more than one filament is used, the filaments may be braided, twisted, entangled, intertwined or arranged in some other multifilament configuration. A particularly useful braid structure for sutures is the spiroid braid structure described in U.S. Pat. Nos. 5.019.093 and 5,059,213 the disclosures of which are incorporated herein by reference.

The coating applied to the monofilament or multifilament structure comprises an oil. Suitable oils include but are not limited to, mineral oil and castor oil.

Castor oil, the fixed oil obtained from the seed of *Ricinus communis,* is a well know and widely available material. Castor oil is a non-drying oil whose chief constituent is ricinolein, a glyceride of ricinoleic acid. It is a transparent, viscous liquid having a specific gravity in the range of 0.945 to 0.965, and iodine value between 83 and 88 and a saponification value between 176 and 182. While castor oil containing no added substances is preferred for use herein, other castor oil products, such as, for example, acetylated castor oil, dehydrated castor oil, hydrogenated castor oil and sulfonated castor oil, can also be used.

Mineral oil is also a well known and widely available material. Mineral oil is a mixture of liquid hydrocarbons obtained form petroleum. Frequently, a stabilizer is added. Mineral oil generally has a specific viscosity between 0.845 and 0.905 and a kinematic viscosity of about 33 to 35 centistokes at 40°.

The oil coating is applied to the monofilament or multifilament in an amount of between about 0.01 to 20 percent by weight based upon the weight of the filament or filaments to which the coating is applied. Preferably, the coating is applied in an amount of from about 0.1 to 10 weight percent. Most preferably, the amount of coating is between about 0.5 and 5 weight percent. The amount of coating applied to the suture may be adequate to coat all surfaces of the suture. Preferably, the amount of coating applied will be that amount sufficient to improve the handling characteristics of the suture, regardless of whether the entire surface of the suture is coated. The term coating as used herein is intended to embrace both full and partial coatings.

The oil coating may be applied by any conventional method. The coating composition may be applied to sutures by dipping the suture in a reservoir of coating composition, moving sutures past a brush or applicator wetted with the composition, or by spraying the composition onto sutures. The amount of coating composition may be varied depending on the construction of the sutures, e.g., the number of filaments and tightness of braid or twist. A less viscous composition will penetrate further into the suture than a more viscous composition. In addition, viscosity of the composition can be adjusted depending on the method of application. For example, a suitable solvent such as, for example, isopropanol can be used to adjust the viscosity of the oil composition prior to application.

The coatings may optionally contain other materials including colorants, such as pigments or dyes, fillers or therapeutic agents, such as antibiotics, growth factors, etc. Depending on the amount of coating present, these optional ingredients may constitute up to about 25 percent by weight of the coating.

The following examples should be considered as illustrative and not as limitations of the present description. The examples show illustrative formulations and the superiority of the present coating composition in enhancing properties of sutures.

### EXAMPLES 1-6

Size 5/0 polypropylene sutures prepared in accordance with the procedures described in the Examples of commonly owned provisional application entitled POLYOLEFIN SUTURES HAVING IMPROVED PROCESSING AND HANDLING CHARACTERISTICS, Serial No. 60/278,686 filed March 26, 2001. The polypropylene from which the sutures were prepared contained 0.3% by weight PEG distearate. Coating compositions containing various amounts of castor oil in isopropanol solvent were prepared as shown in Table 1, below. The coating was applied using a spin finish applicator. The castor oil coating was applied after stretching but before annealing. The isopropanol evaporated during annealing.

**Table 1**

| Example # | % Castor oil |
|---|---|
| 1 | 20 |
| 2 | 10 |
| 3 | 5 |
| 4 | 2.5 |
| 5 | 1.25 |
| 6 | 0.625 |

The sutures coated with solutions containing at least 2.5% castor oil were found to exhibit improved fray resistance compared to polypropylene/PEG distearate sutures prepared under identical conditions but without application of the castor oil containing compositions. The sutures coated with composition containing castor oil also possessed a lower coefficient of friction, generally below about 0.2, compared to polypropylene/PEG distearate sutures prepared under identical conditions but without application of the castor oil containing compositions.

It will be understood that various modifications may be made to the embodiments disclosed herein. Therefore, the above description should not be construed as limiting, but merely as exemplifications within the scope and spirit of the claims appended hereto.

## Claims

1. A suture comprising:
a filament made from a synthetic, non-absorbable polymer composition ; and
a coating, the coating being made from a composition containing an oil selected from the group consisting of castor oil and mineral oil.

2. A suture as in claim 1 wherein the synthetic, non-absorbable polymer composition comprises polypropylene.

3. A suture as in claim 1 wherein the synthetic, non-absorbable polymer composition comprises polyproplene and a fatty acid distearate.

4. A suture as in claim 1 wherein the coating is made from a composition containing castor oil.

5. A suture comprising:
a filament made from a composition comprising polyproplene and polyethylene glycol distearate; and
a coating, the coating being made from a composition containing castor oil in a solvent.

6. A suture as in claim 5 wherein the solvent is isopropanol.

7. A method of improving the fray resistance of a suture made from a polypropylene-containing composition, the method comprising:
applying a coating to the suture, the coating being made from a composition containing an oil selected from the group consisting of castor oil and mineral oil.

8. A method in accordance with claim 7 wherein the step of applying a coating comprises applying a coating made from a composition containing castor oil in a solvent.

9. A method of making a suture comprising:
providing a composition comprising polypropylene;
melt spinning the composition to form a filament;
stretching the filament;
applying a coating to the filament, the coating being made from a composition containing an oil selected from the group consisting of castor oil and mineral oil; and
annealing the coated filament.

10. A method as in claim 9 wherein the step of providing a composition containing polypropylene comprises providing a composition containing polypropylene and polyethylene glycol distearate.

11. A method as in claim 9 wherein the step of applying a coating to the filament comprises applying a coating made from a composition containing castor oil in a solvent.
